(19) Europäisches Patentamt
European Patent Office
Office européen des brevets

(11) **EP 0 799 021 B1**

(12) **EUROPÄISCHE PATENTSCHRIFT**

(45) Veröffentlichungstag und Bekanntmachung des Hinweises auf die Patenterteilung:
**01.09.1999 Patentblatt 1999/35**

(51) Int. Cl.$^6$: **A61K 7/48**, A61K 7/00

(21) Anmeldenummer: 95941588.6

(86) Internationale Anmeldenummer:
**PCT/DE95/01801**

(22) Anmeldetag: **06.12.1995**

(87) Internationale Veröffentlichungsnummer:
**WO 96/17588 (13.06.1996 Gazette 1996/27)**

(54) **KOSMETISCHE KAOLINHALTIGE ZUBEREITUNG**

COSMETIC KAOLIN-CONTAINING PREPARATION

PREPARATION COSMETIQUE CONTENANT DU KAOLIN

(84) Benannte Vertragsstaaten:
**AT BE CH DE DK ES FR GB GR IE IT LI LU MC NL PT SE**

(30) Priorität: **07.12.1994 DE 4445064**

(43) Veröffentlichungstag der Anmeldung:
**08.10.1997 Patentblatt 1997/41**

(73) Patentinhaber:
**LANCASTER GROUP GmbH
55116 Mainz (DE)**

(72) Erfinder:
• **GOLZ, Karin
MC-98000 Monaco (MC)**

• **ZASTROW, Leonhard
MC-98000 Monaco (MC)**
• **STANZL, Klaus
White Plains, NY 10605 (US)**
• **BRAUNAGEL, Alfred
D-55128 Mainz (DE)**

(74) Vertreter:
**Walter, Wolf-Jürgen et al
Patentanwälte Felke & Walter
Normannenstrasse 1-2
10367 Berlin (DE)**

(56) Entgegenhaltungen:
**US-A- 5 182 103**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue kaolinhaltige Zubereitungen, die in der Kosmetik in Form von Emulsionen oder Gelen eingesetzt werden können.

Es ist bekannt, Kaolin bestimmten dermatologischen Zubereitungen zuzusetzen und damit eine gewisse entzündungswidrige Wirkung zu erzielen. Für die Kosmetik sind bisher nur Kaolinzusätze bis zu maximal 2 Gewichts-% bei gleichbleibend gutem Hautgefühl möglich gewesen, da über diese Grenze hinaus die Emulsion zu starken Verklumpungen neigte und ein höherer Zusatz zur Verstumpfung der Emulsion führte, was im Kosmetikbereich nicht tolerierbar war.

Aus der EP-B-406657 sind plättchenförmige Substrate bestimmter Größe aus z. B. Zeolith, Glimmer oder Glas bekannt, die einen Anteil sphärischer Teilchen zur Verhinderung der Agglomeration enthalten.

Aufgabe der vorliegenden Erfindung ist es, neue Zubereitungen für die Kosmetik zu entwickeln, die neben der pflegenden Wirkung eine im erhöhten Maße entzündungswidrige Wirksamkeit haben und zugleich ein besonders angenehmes Hautgefühl vermitteln.

Erfindungsgemäß besteht die kosmetische kaolinhaltige Zubereitung aus

(a) einer Mischung von weißem Kaolin, der einen hohen Kaolinitgehalt hat, vorzugsweise von mehr als 85 Gew.-%, insbesondere von mehr als 95 Gew.-%, modifiziert mit sphärischen anorganischen Teilchen, die eine Teilchengröße von kleiner als 5 $\mu$m haben, in einem Anteil der sphärischen Teilchen an der Kaolinmischung von 0,5 bis 10 Gew.-%, und

(b) einer pflegenden kosmetischen Zusammensetzung der Gruppe Emulsionen und Emulsionsgrundlagen für Lotionen, Cremes, Masken; Gele und Gelgrundlagen für Lotionen, Cremes, Masken; wobei der Anteil des Gemisches Kaolin/sphärische Teilchen, bezogen auf die Gesamtzusammensetzung, im Bereich von mehr als 2 bis 65 Gew.-% liegt.;

und wobei die Viskosität der Gesamtzusammensetzung mit dem Anteil Kaolin/sphärische Teilchen um 2000 cps bis mehr als 15.000 cps niedriger liegt als eine gleiche Zusammensetzung mit nur Kaolin ohne sphärische Teilchen, wobei die Viskositätsdifferenz mit höherem Anteil Kaolin/sphärische Teilchen steigt.

Ein erfindungsgemäßes Merkmal besteht weiterhin darin, daß die genannten kosmetischen Fumulierungen ein besonders angenehmes Hautgefühl und eine nichtklebrige und nichtplastische Konsistenz haben.

Besonders vorteilhaft sind solche Kaolingemische, denen der Anteil der sphärischen Teilchen 0,5 bis 5 Gew.-% beträgt. Weiterhin ist vorteilhaft, wenn die Teilchengröße der sphärischen Teilchen im Bereich von 0,1 bis 3 $\mu$m liegt, insbesondere im Bereich von 0,1 bis 1 $\mu$m.

Der Anteil des modifizierten Kaolingemisches (Gemisch Kaolin/sphärische Teilchen) ist im gesamten Bereich von etwa 2 bis etwa 65 Gew.-% einsetzbar, jedoch ist es besonders vorteilhaft, wenn das modifizierte Kaolingemisch im Bereich von 5 bis 30 Gew.-% liegt, obwohl auch noch höhere Gehalte insbesondere bei Pudern möglich sind.

Überraschenderweise kann durch Formulierung eines Kaolingemisches mit geringem Anteil an sphärischen anorganischen Teilchen, wie z. B. amorphem Siliciumdioxid, nicht nur eine Verbesserung der Agglomerierung innerhalb der Kaolinsuspension erreicht werden, sondern es wird die Einarbeitung des Kaolins in eine kosmetische Zusammensetzung wie z. B. eine Emulsion, in einem so hohen Anteil möglich, wie dies vom Fachmann nicht zu erwarten war. Das eingesetzte Kaolin, das einen Durchmesser der Plättchen im Bereich von 0,2-1 $\mu$m hat, bildet mit den nahezu gleich großen $SiO_2$-Teilchen keine übergeordnete Makrostruktur aus, wodurch der bekannte Zustand der Plastizität von Tonen vermieden wird, und eine niedriger viskose Dispersion erhalten wird gegenüber einem üblichen Gemisch aus Kaolin und wäßrigen kosmetischen Zusatzstoffen, die zu Gemischen mit Plastizitätserscheinungen führen.

Weiterhin wird synergistisch, das "feeling" der Gesamtzubereitung auf der Haut durch ein bisher nicht erreichtes Weichheitsgefühl erhöht, damit der kosmetische Effekt vergleichbarer Produkte übertroffen und zugleich eine wesentliche entzündungswidrige Wirksamkeit erzielt.

Ein weiterer überraschender Effekt ist bei Gelen darin zu sehen, daß bereits die Einbeziehung von 2,5 bis 3 Gew.-% des modifizierten Kaolins in eine Gelmischung die Klebrigkeit des Gels stark verringert und die schlechte Spreitbarkeit bestimmter Gele wesentlich erhöht. Dadurch wird der Anwendungsbereich von Gelen in der Kosmetik erweitert.

Die Viskosität von erfindungsgemäßen kosmetischen Formulierungen z.B. als kosmetische Maske liegt beispielsweise bei einem Anteil des Gemisches Kaolin/sphärische Teilchen an der Gesamtzusammensetzung von 2,5 Gew-% um etwa 2000 cps niedriger als bei einer gleichen Mischung ohne die sphärischen Teilchen; sie liegt bei 30 Gew-% um etwa 15.000 cps niedriger und bei 45 Gew-% um wesentlich mehr als 15.000 cps niedriger. Die Messung erfolgte mit einem Broockfield Viskosimeter RVT/DVII mit den entsprechenden Spindeln C, resp. D, resp. E, resp. F. Mit Spindel F war die Viskosität der Mischung mit reinem Kaolin ohne sphärische Teilchen bei 45 Gew-% Zusatz über 65.000 cps und nicht mehr meßbar, während sie beim Gemisch Kaolin/sphärische Teilchen (45 Ge-%) etwa 52.000 cps betrug.

Vorteilhaft für die erfindungsgemäßen zubereitungen kann es sein, daß die Zubereitung das modifizierte Kaolingemisch verkapselt in üblichen Liposomen oder - noch günstiger - in asymmetrischen lamellaren Aggregaten enthält, wobei diese Aggregate aus Phospholipiden und mit Sauerstoff beladenes Fluorcarbon oder Fluorcarbongemisch bestehen und deren Gehalt an Fluorcarbon im Bereich von 0,2 bis 100 % Gewicht/Volumen liegt, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen.

Die Aggregate können auch zusätzlich allein nur mit Sauerstzoff beladen in der kosmetischen Zubereitung vorliegen.

Diese Aggregate sind Sauerstoffträger und ermöglichen ein Penetrieren des Sauerstoffs in die Haut und damit eine bessere Versorgung der Haut mit Sauerstoff. Die Herstellung dieser Aggregate erfolgt durch Hochdruckhomogenisierung von Phospholipiden, wie Sojalecithin und Eilecithin oder synthetischen Phospholipiden oder teilhydrierten Phospholipiden, die einen Phosphatidylcholingehalt von mehr als 30 Gew.-% bis 99 Gew.-% haben, mit perfluorierten oder hochfluorierten Kohlenstoffverbindungen oder Gemischen davon, die in der Lage sind, Gase, wie Sauerstoff und Kohlendioxid zu transportieren. Darin können neben Phosphatidylcholin auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Im Unterschied zu den bekannten wäßrigen Liposomen (Vesikel) tragen diese Phospholipid-stabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und gegebenenfalls ein sich daran anschließender Aufbau von Bilayer-Schichten. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 30 bis 99 % in Verbindung mit Lysolecithinen der Konzentration von 0,1 bis 10 % und/oder geladenen Phospholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert. Wesentlich für den Einsatz der Fluorcarbon-Aggregate ist die Hautpenetrierung in Abhängigkeit von der kritischen Löslichkeitstemperatur der aus gewählten Fluorcarbone oder Fluorcarbongemische (für den Einsatz asymmetrischer lamellarer Aggregate s.a. DE-B-42 21 255).

Der Anteil der mit der modifizierten kaolinhaltigen Mischung beladenen Aggregate kann im Bereich von 5 bis 60 Gew.-% liegen, bezogen auf die Gesamtzubereitung, und liegt vorteilhaft im Bereich von 10 bis 50 Gew.-%, insbesondere im Bereich von 15 bis 30 Gew.-%.

Wie bereits ausgeführt, können auch übliche Liposome als Transportsystem für das modifizierte kaolinhaltige Gemisch innerhalb der erfindungsgemäßen Zubereitung eingesetzt werden. Liposome sind vollständig geschlossene Lipid-Bilayer-Membranen, die ein wäßriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wäßrige Schicht getrennt ist). Die Bilayer besteht aus zwei Lipid-Monolayern, die einen hydrophoben "Schwanz"-Bereich und einen hydrophilen "Kopf"-Bereich haben. Die Struktur der Membran-Bilayer ist so, daß die hydrophoben (unpolaren) "Schwänze" der Lipidmonolayer sich in Richtung des Zentrums der Bilayer orientieren, während sich die hydrophilen "Köpfe" in Richtung der wäßrigen Phase orientieren.

Die Herstellung von Liposomen, aus gesättigten und ungesättigten Lipiden, ist in sehr vielen Patenten beschrieben worden, ebenso deren Einsatz als Transportsystem. Die Einarbeitung des modifizierten kaolinhaltigen Gemisches kann auf übliche Weise erfolgen.

Erfindungsgemäß bevorzugt sind solche kaolinhaltige Zubereitungen, die ein Produkt des schonenden Aufschlusses durch Ultraschall und/oder Hochdruckhomogenisierung von Suspensionen oder Dispersionen von Zellen pflanzlicher Stoffe, Bakterien oder Hefen enthalten. Als pflanzliche Stoffe können solche eingesetzt werde, die bisher in der Kosmetik vorteilhaft verwendet wurden, wie Kamille, Aloe vera usw., jedoch auch solche Produkte, wie die Rinde des mexikanischen Hautbaumes (Mimosa tenuiflora), mit der ein besonders entzündungshemmendes sauerstoffreiches Produkt erhalten wird.

Als Hefen können Bäckerhefe, Weinhefe, Bierhefe und andere verwendet werden.

Ein besonders vorteilhaftes Aufschlußprodukt rührt aus einem Ultraschallaufschluß mit einer Ultraschall-Durchflußzelle gemäß der DE 42 41 154 her, bei der die Synotrode zu $\frac{1}{2}$ bis $\frac{2}{3}$ ihrer Länge in die Durchflußzelle hineinragt, der Winkel der Synotrode im Beschallungsgefäß im Bereich von 80,5 bis 88,5 ° liegt, das Verhältnis der Eintauchlänge der Synotrode (in mm) zum Beschallungsvolumen (in ml) auf einen Wert im Bereich von 1:1,1 bis 1:20 eingestellt ist und das Verhältnis von Eintauchlänge der Synotrode (in mm) zu dem Feststoffanteil des zu beschallenden Mediums (in Masse-%) im Bereich von 1:0,02 bis 1:2,2 liegt.

Die Wirkung der bevorzugten Kombination von asymmetrischen lamellaren Aggregaten, beladen mit dem modifizierten Kaolingemisch und den Aufschlußprodukten pflanzlicher und Hefezellen in einer erfindungsgemäßen kosmetischen Zubereitung ist besonders ausdrucksvoll beim Aufschluß von Superoxiddismutase-reichen Hefen, die ein hohen Gehalt an Superoxiddismutase (SOD) liefern. Da SOD als Radikalfänger in der Haut wirkt und die Reaktion

$$2\,O_2^- + 2\,H^+ \rightarrow H_2O_2 + O_2$$

katalysiert, ist es per se ein besonders vorteilhafter Bestandteil in kosmetischen oder dermatologischen Präparaten. Es können jedoch auch andere Radikalfänger verwendet werden, die sich zur Bindung freier Sauerstoffradikale eignen, beispielsweise Vitamin E.

Geeignete Formen der kosmetischen Zubereitung sind Emulsionen, vorzugsweise kosmetische Masken, insbesondere Gesichtsmasken. Bei derartigen Masken liegt der Gehalt der modifizierten Kaolinsuspension vorteilhaft im Bereich von 3 bis 30 Gew.-%, vorzugsweise 5 bis 20 Gew.-%. Daneben kann die Emulsion übliche Bestandteile, wie Emulgatoren, Parfümöle, Schutzmittel sowie weitere pflegende Bestandteile enthalten.

Ebenso bevorzugt sind Gele wegen der bereits genannten verbesserten Spreitbarkeit, wobei der Anteil des modifizierten Kaolins vozugsweise im Bereich von 5 bis 15 Gew.-% liegt.

Das Gemisch Kaolin/sphärische Teilchen kann auch in Pudern eingesetzt werden; ebenso in pharmazeutischen Zubereitungen.

Die Herstellung der Zubereitung erfolgt im allgemeinen, indem eine Dispersion von gereinigtem weißem Kaolin mit hohem Kaolinitanteil mit einer Dispersion der sphärischen anorganischen Teilchen bei Umgebungstemperatur vermischt wird. Als Dispergiermittel wird vorzugsweise Wasser eingesetzt. Danach wird die Zubereitung mit der pflegenden kosmetischen Zusammensetzung, sofern diese als Emulsion vorliegt, in einem Emulgator vermischt.

Nach Vermischung der Kaolindispersion mit der Dispersion sphärischer Teilchen, insbesondere mit einer $SiO_2$-Dispersion, kann eine Sprühtrocknung erfolgen, und das trockene Produkt kann direkt in die Emulsion eingearbeitet oder gegebenenfalls zuvor in Liposome oder asymmetrische lamellare Aggregate aufgenommen und dann in die Emulsion eingearbeitet werden.

Die Erfindung soll nachstehend durch Beispiele näher erläutert werden. Alle Prozentangaben sind Gewichtsprozente.

Beispiel 1

Herstellung von modifiziertem Kaolin (1)

30 l einer Aufschlämmung von 4 kg weißem Kaolin mit einem Kaolinitgehalt von 96 % in Wasser/Ethanol wurde gerührt und nacheinander innerhalb von 30 Minuten zu 40 l einer Aufschlämmung von 4,5 kg monodispersem Siliciumdioxid gegeben und eine weitere Stunde gerührt. Danach wurde das Produkt auf einem üblichen Sprühturm sprühgetrocknet. Man erhielt ein Produkt, das direkt für eine Verarbeitbarkeit in kosmetischen Produkten geeignet war.

Beispiel 2

Herstellung von modifiziertem Kaolin (2)

Es wurde wie im Beispiel 1 gearbeitet, mit Ausnahme dessen, daß als Lösungsmittel ein mehrwertiger Alkohol wie Ethylenglycol eingesetzt wurde und die Menge an Kaolin 3,5 kg betrug.

Beispiel 3

Kozmetische Maske (1)

Es wurden separat 5 Phasen hergestellt, die nacheinander miteinander vermischt wurden, teilweise unter intensiver Homogenisierung. Dabei wurden die Phasen A bis D bei erhöhter Temperatur (40 bis 70 °C) und die Phase D am Ende bei Umgebungstemperatur eingearbeitet.

| Phase A | |
|---|---|
| Glycerylstearat | 4,1 % |
| Stearinsäure | 1,8 % |
| Cetylalkohol | 1,8 % |
| **Phase B** | |
| dest. Wasser | q.s. |
| Carbomer | 0,3 % |
| Propylenglycol | 2,5 % |
| Konservierungsmittel | 0,3 % |
| **Phase C** | |
| Triethanolamin (TEA) | 0,3 % |
| **Phase D** | |
| modifizierter Kaolin | 30 % |
| Wirkstoffkomplex Kamille | 3 % |
| **Phase E** | |
| Parfümöl | 0,5 % |

Beispiel 4

Kosmetisches Gel

Das Carbomer wurde mit Wasser homogenisiert und dem Homogenisat nacheinander TEA, Konservierungsmmittel und das modifizierte Kaolin unter Rühren zugesetzt.

| Carbomer | 1,0 % |
|---|---|
| TEA | 0,8 % |
| Wasser | q.s. |
| modifiziertes Kaolin | 2,5 % |
| Konservierungsmittel | 0,3 % |

Beispiel 5

Kosmetischer Puder

Die nachfolgend genannten Bestandteile wurden in der angegebenen Reihenfolge miteinander vermischt.

| modifizierter Kaolin | 65 % |
|---|---|
| Magnesiumstearat | 5 % |
| Seidenprotein | 10 % |

(fortgesetzt)

| Zinkoxid | 5 % |
|---|---|
| Mais/Reisprotein | 10 % |
| Farbe | ca. 5 % |

Beispiel 6

Kaolin-beladene Liposome

Es wurden mit modifiziertem Kaolin (gemäß Beispiel 1 und 2) beladene Liposome wie folgt hergestellt. Ein Phospholipid wurde in eine Wasser-Kaolin-Suspension eingetragen, wobei der Kaolin gemäß Beispiel 2 hergestellt worden war. Nach guter Homogenisierung wurde unter Rühren Ethanol hinzugegeben und weiter homogenisiert.

| Phospholipid | 15 % |
|---|---|
| modifizierter Kaolin | 7 % |
| Wasser | q.s. |

Beispiel 7

Kaolin-beladene Aggregate

Es wurden mit modifiziertem Kaolin (gemäß Beispiel 1 und 2) beladene asymmetrische lamellare Aggregate wie folgt hergestellt. In Perfluordekalin, vermischt mit Glycerin und Propylenglycol wurde modifiziertes Kaolin unter guter Homogenisierung eingetragen. Ein Phospholipid mit einem Phosphatidylcholingehalt von 50 % wurde in dieses Homogenisat eingerührt und dann Wasser hinzugegeben. Nach guter Homogenisierung standen die mit modifiziertem Kaolin beladenen Aggregate für die weitere Verwendung zur Verfügung.

| Phospholipid | 25 % |
|---|---|
| modifizierter Kaolin | 4 % |
| Florcarbon (Perfluordekalin) | 50 % |
| Glycerin | 3,5 % |
| Propylenglycol | 5,0 % |
| Wasser | q.s. |

Beispiel 8

Es wurde wie im Beispiel 7 gearbeitet, mit Ausnahme dessen, daß der Anteil an modifiziertem Kaolin 10 % betrug.

Beispiel 9

Es wurde wie im Beispiel 7 gearbeitet, wobei zusätzlich zum modifizierten Kaolin mittels des oben beschriebenen Ultraschall-Aufschlußverfahrens aufgeschlossene Bäckerhefe in Form des Zentrifugatüberstandes in das Fluorcarbongemisch homogenisiert wurden.

EP 0 799 021 B1

| | |
|---|---|
| Phospholipid | 20 % |
| modifizierter Kaolin | 5 % |
| Glycerin | 3,5 % |
| Propylenglycol | 5 % |
| Hefe-Aufschluß | 16 % |
| Perfluordekalin | 40 % |

Beispiel 10

Es wurde wie im Beispiel 9 gearbeitet, mit Ausnahme dessen, daß ein Aufschlußprodukt der Rinde des mexikanischen Hautbaumes (Mimosa tenuiflora) in einem Anteil von 19,5 % eingebracht wurde.

Beispiel 11

Sonnenschutzcreme

Nach Homogenisierung bei erhöhter Temperatur der Phasen A und B wurden beide miteinander vermischt und dabei gut homogenisiert. Anschließend wurde die Phase C bei etwa 30 bis 40 °C hinzugemischt.

| Phase A | |
|---|---|
| Sorbitansesquioleat | 5,0 % |
| Cetylalkohol | 4,5 % |
| Stearylalkohol | 3,5 % |
| Titandioxid | 3,6 % |
| **Phase B** | |
| Propylenglycol | 2,0 % |
| Wasser | q.s. |
| Glycerin | 1,0 % |
| Titandioxid | 1,9 % |
| **Phase C** | |
| Konservierungsmittel | 0,3 % |
| Parfümöl | 0,4 % |
| mit modifiziertem Kaolin beladene asymmetrische lamellare Aggregate gemäß Beispiel 9 | 30,0 % |

Beispiel 12 und 13

After Sun Gel

In das fertige Gel wurden unter Rühren nacheinander die nach Beispiel 9 bzw. 10 hergestellten, mit modifiziertem Kaolin und einem Hefe- bzw. Hautbaumaufschluß beladenen asymmetrischen lamellaren Aggregate, Parfümöl und Konservierungsmittel bei Umgebungstemperatur eingetragen.

| | |
|---|---|
| Polyacrylsäure (MG ca. 4 000 000) | 1,0 % |
| Hydroxyethylcellulose | 0,3 % |
| Propylenglycol | 3,0 % |
| Benzoesäure | 0,3 % |
| modifizierter Kaolin mit Hefe/ Hautbaumaufschluß in Aggregaten | 25,0 % |
| Parfümöl | 0,3 % |
| Konservierungsmittel | 0,3 % |
| Wasser | q.s. |

Beispiel 14

Kosmetische Maske (2)

Es wurde wie im Beispiel 3 gearbeitet, mit Ausnahme dessen, daß die Menge an modifiziertem Kaolin 12 % betrug.

Beispiel 15

Pharmazeutische Salbe

Nach Homogenisierung und homogener Vermischung der Phasen A und B bei erhöhter Temperatur (etwa 65 °C) wurde die Phase C bei 30 bis 40 °C zugegeben.

| Phase A | |
|---|---|
| Lanolin | 5,0 % |
| Cetylalkohol | 2,0 % |
| Cetylalkohol und PEG-40 Castor Oil im Verhältnis 1:1 | 3,0 % |
| Hexyllaurat | 1,5 % |
| **Phase B** | |
| Wasser | q.s. |
| Glycerin | 2,0 % |
| Propylalkohol | 2,0 % |
| modifizierter Kaolin | 45,0 % |
| **Phase C** | |
| Konservierungsmittel | 0,3 % |

Vergleichsbeispiel 1

Bei einer Gruppe von 10 weiblichen Testpersonen wurde eine kosmetische Hals-Dekollete-Maske aufgebracht. Die Maske A entsprach der des erfindungsgemäßen Beispiels 14. Die Maske B enthielt normalen Kaolin mit einem Kaolinitgehalt von 97 %, wobei der Kaolingehalt infolge Schwierigkeiten bei der Verarbeitung auf 8 % gesenkt werden mußte. Die Bewertung durch die Testpersonen erfolgte nach einer Skala

1 = sehr angenehmes Hautgefühl

2 = angenehmes Hautgefühl

3 = unangenehmes Hautgefühl

4 = sehr unangenehmes Hautgefühl

| | Bewertung in % | | | |
|---|---|---|---|---|
| | 1 | 2 | 3 | 4 |
| Maske A | 90 | 10 | 0 | 0 |
| Maske B | 0 | 0 | 80 | 20 |

Daraus ist die deutliche Überlegenheit der erfindungsgemäßen Maske zu entnehmen, was das Hautgefühl betrifft. Eine Gegenüberstellung der entzündungswidrigen Wirksamkeit konnte wegen der unterschiedlichen Gehalte an Kaolin nicht erfolgen.

**Patentansprüche**

1. Kosmetische kaolinhaltige Zubereitung, dadurch gekennzeichnet, daß sie als Lotion, Maske, Creme oder Gel mit besonders angenehmem Hautgefühl mit nichtklebriger und nichtplastischer Konsistenz vorliegt, bestehend aus einer Mischung aus weißem Kaolin mit hohem Kaolinitgehalt und sphärischen anorganischen Teilchen aus Siliciumdioxid oder Titanoxid, die eine Teilchengröße von kleiner als 5 $\mu$m haben, in einem Anteil der sphärischen Teilchen an der Kaolinmischung von 0,5 bis 10 Gew.-%, dispergiert in einer wäßrigen kosmetischen Zusammensetzung der Gruppe Emulsionen und Emulsionsgrundlagen für Lotionen, Cremes, Masken; Gele und Gelgrundlagen für Lotionen, Cremes, Masken;
der Anteil der Mischung Kaolin/sphärische Teilchen liegt, bezogen auf die Gesamtzusammensetzung, im Bereich von mehr als 2 bis 65 Gew.-%;
die Viskosität der Gesamtzusammensetzung mit einem Anteil Kaolin/sphärische Teilchen liegt um 2000 cps bis mehr als 15.000 cps niedriger als eine gleiche Zusammensetzung mit nur Kaolin ohne sphärische Teilchen, wobei die Viskositätsdifferenz mit höherem Anteil Kaolin/sphärische Teilchen steigt.

2. Kosmetische kaolinhaltige Zubereitung nach Anspruch 1, dadurch gekennzeichnet, daß der Anteil der sphärischen Teilchen an der Kaolinmischung 0,5 bis 5 Gew.-% beträgt.

3. Kosmetische kaolinhaltige Zubereitung nach Anspruch 1, dadurch gekennzeichnet, daß die Teilchengröße der sphärischen Teilchen im Bereich von 0,1 bis 3 $\mu$m liegt, insbesondere im Bereich von 0,1 bis 1 $\mu$m.

4. Kosmetische kaolinhaltige Zubereitung nach Anspruch 1, dadurch gekennzeichnet, daß der Anteil der Mischung Kaolin/sphärische Teilchen 5 bis 30 Gew.-% beträgt.

5. Kosmetische kaolinhaltige Zubereitung nach Anspruch 1, dadurch gekennzeichnet, daß sie die Mischung Kaolin/sphärische Teilchen in asymmetrischen lamellaren Aggregaten enthält, die aus Phospholipiden und mit Sauerstoff beladenem Fluorcarbon oder Fluorcarbongemisch bestehen, wobei der Anteil an Fluorcarbon im Bereich von 0,2 bis 100 % Gewicht/Volumen liegt, mit einem Phosphatidylcholingehalt der Lipidfraktion von 30 bis 99 Gewichts-%, und wobei die Aggregate eine Hautpenetrierung in Abhängigkeit von der kritischen Löslichkeitstemperatur der Fluorcarbone aufweisen.

6. Kosmetische kaolinhaltige Zubereitung nach Anspruch 5, dadurch gekennzeichnet, daß zusätzlich allein mit Sauerstoff beladene asymmetrische lamellare Aggregate im Gemisch enthaltenm sind.

7. Kosmetische kaolinhaltige Zubereitung nach Anspruch 5, dadurch gekennzeichnet, daß die Aggregate neben der Mischung Kaolin/sphärische Teilchen ein Produkt des schonenden Aufschlusses durch Ultraschall und/oder Hochdruckhomogenisierung von Suspensionen oder Dispersionen von Zellen pflanzlicher Stoffe, Bakterien oder Hefen enthalten.

8. Verwendung einer kaolinhaltigen Zubereitung aus einer Mischung aus weißem Kaolin mit hohem Kaolinitgehalt und sphärischen anorganischen Teilchen, die eine Teilchengröße von < 5 μm haben, bei einem Anteil der sphärischen Teilchen an der Kaolinmischung von 0,5 bis 10 Gew.-%, in einer kosmetischen Maske, einem kosmetischen Gel oder einer kosmetischen Creme, wobei der Anteil der Mischung Kaolin/sphärische Teilchen, bezogen auf die Gesamtzusammensetzung, im Bereich von 2,5 bis 30 Gew.-% liegt.

## Claims

1. Cosmetic preparation containing kaolin, characterized by its availability as a lotion, mask, cream or gel with a particularly pleasant sensation on the skin and a non-sticky, non-plasticized consistency, consisting of a mixture of white kaolin with a high proportion of kaolinite and spherical inorganic particles of silicon dioxide or titanium dioxide with a particle size of less than 5 μm and a proportion of spherical particles in the kaolin mixture of 0.5 to 10 % by weight, dispersed in an aqueous cosmetic preparation of the emulsion and emulsion base group for lotions, creams and masks as well as gels and gel bases for lotions, creams and masks;
the proportion of the kaolin/spherical particle mixture falls within the range of over 2 to 65 % by weight of the composition as a whole;
the viscosity of the composition with the share of kaolin/spherical particles is around 2000 to over 15,000 cps lower than the same composition with kaolin only and without spherical particles, with the difference in viscosity increasing with the proportion of kaolin/spherical particles.

2. Cosmetic preparation containing kaolin according to Claim 1, characterized by the proportion of spherical particles of 0.5 to 5 % by weight of the kaolin mixture.

3. Cosmetic preparation containing kaolin according to Claim 1, characterized by the size of the spherical particles which lies in the range of 0.1 to 3 μm, particularly in the range of 0.1 to 1 μm.

4. Cosmetic preparation containing kaolin according to Claim 1, characterized by the proportion of the kaolin/spherical particle mixture which amounts to 5 to 30 % by weight.

5. Cosmetic preparation containing kaolin according to Claim 1, characterized by the containment of the kaolin/spherical particle mixture in asymmetric lamellar aggregates consisting of phospholipids and oxygen-charged fluorocarbon or fluorocarbon mixture in which the proportion of fluorocarbon lies in the range from 0.2 to 100 % by weight/volume, with a phosphatidylcholine content of the lipid group of 30 to 99 % by weight, and in which the aggregates demonstrate skin penetration dependent on the critical solubility temperature of the fluorocarbons.

6. Cosmetic preparation containing kaolin according to claim 5, characterized by the additional content of asymmetric lamellar aggregates charged solely with oxygen in the mixture.

7. Cosmetic preparation containing kaolin according to Claim 5, characterized in that the aggregates, in addition to the kaolin/spherical particle mixture, contain a product of the cautious ultrasonification and/or high-pressure homogenization of suspensions or dispersions of plant cells, bacteria or yeasts.

8. Use of a preparation containing kaolin consisting of a mixture of white kaolin with a high proportion of kaolinite and spherical inorganic particles of a particle size of less than 5 μm, with a proportion of spherical particles in the kaolin mixture of 0.5 to 10 % by weight in a cosmetic mask, cosmetic gel or cosmetic cream, in which the proportion of the kaolin/spherical particle mixture lies in the range of 2.5 to 30 % by weight of the total preparation.

## Revendications

1. Préparation cosmétique contenant du kaolin, caractérisée en ce qu'elle se présente sous forme de lotion, masque, crème ou gel avec une sensation particulièrement agréable pour la peau et une consistance non-collante et non-plastique, constituée d'un mélange de kaolin blanc à haute teneur en kaolinite et de particulies sphériquesminérales de dioxyde de silicium ou d'oxyde de titane, qui présentent une granularité inférieure à 5 μm, dans une proportion des particules sphériques dans le mélange de 0,5 à 10 % en poids, dispersée dans une composition cosmétique aqueuse du groupe des émulsions et bases d'émulsions pour lotions, crèmes, masques; gels et bases de gels pour lotions, crèmes, masques;
la proportion du mélange kaolin/particules sphériques se situe dans l'intervalle de plus de 2 à 65 % en poids, rapporté à la composition globale;
la viscosité de la composition globale avec une proportion de kaolin/particules sphériques est inférieure de 2000 cps à plus de 15000 cps à celle d'une même composition comprenant seulement du kaolin sans particules sphériques, la différence de viscosité augmentant avec une proportion supérieure de kaolin/particules sphériques.

2. Préparation cosmétique contenant du kaolin selon la revendication 1, caractérisé en ce que la proportion des particules sphériques dans le mélange de kaolin est de 0,5 à 5 % en poids.

3. Préparation cosmétique contenant du kaolin selon la revendication 1, caractérisée en ce que la granularité des particules sphériques se situe dans l'intervalle de 0,1 à 3 $\mu$m, en particulier dans l'intervalle de 0,1 à 1 $\mu$m.

4. Préparation cosmétique contenant du kaolin selon la revendication 1, caractérisée en ce que la proportion du mélange kaolin/particules sphériques est de 5 à 30 % en poids.

5. Préparation cosmétique contenant du kaolin selon la revendication 1, caractérisée en ce qu'elle contient le mélange kaolin/particules sphériques dans des agrégats lamellaires asymétriques composés de phospholipides et de fluorocarbone ou mélange de fluorocarbones chargés en oxygène, la proportion du fluorocarbone dans le mélange se situant dans l'intervalle de 0,2 à 100 % en poids/volume, avec une teneur en phosphatidylcholine de la fraction lipide de 30 à 99 % en poids, et les agrégats présentant une pénétration dans la peau qui est fonction de la température de solubilité critique des fluorocarbones.

6. Préparation cosmétique contenant du kaolin selon la revendication 5, caractérisée en ce qu'en plus, seuls des agrégats lamellaires asymétriques chargés en oxygène sont contenus dans le mélange.

7. Préparation cosmétique contenant du kaolin selon la revendication 5, caractérisée en ce qu'en plus du mélange kaolin/particules sphériques, les agrégats contiennent un produit de la désintégration délicate par homogénéisation par ultrasons et/ou à haute pression de suspensions ou de dispersions de cellules de matières végétales, de bactéries ou de levures.

8. Utilisation d'une préparation constituée d'un mélange de kaolin blanc à teneur élevée en kaolinite et de particules minérales sphériques, qui présentent une granularité < 5 $\mu$m, avec une proportion des particules sphériques dans le mélange de kaolin de 0,5 à 10 % en poids, dans un masque cosmétique, dans un gel cosmétique ou dans une crème cosmétique, la proportion du mélange kaolin/particules sphériques se situant dans l'intervalle de 2,5 à 30 % en poids.